# EUROPEAN PATENT APPLICATION

(11) **EP 4 589 355 A1**
(43) Date of publication of application: **23.07.2025**
(21) Application number: 24152501.3
(22) Date of filing: 18.01.2024
(51) Int. Cl.: G02B 21/00, A61B 5/00, A61B 90/20, A61B 90/00, G16H 30/20

(54) **SURGICAL IMAGING SYSTEM AND CORRESPONDING SYSTEM, METHOD AND COMPUTER PROGRAM**

(71) Applicant: Leica Instruments (Singapore) Pte. Ltd., Singapore 608924 (SG)
(72) Inventor: THEMELIS, George, 608924 Singapore (SG)
(74) Representative: 2SPL Patentanwälte PartG mbB

(57) **Abstract**

Some aspects of the present disclosure relate to a system (110) for a surgical imaging system (100) that is configured to obtain sensor data from a sensor (150, 122) of the surgical imaging system, the sensor data representing a distance between an objective (124) of a surgical imaging device (120) of the surgical imaging system and a plurality of points of a surface (10) of a surgical site being imaged by the surgical imaging system, determine a three-dimensional profile of the surface of the surgical site based on the sensor data, determine an intersection between the surface of the surgical site and a three-dimensional pre-operative scan of the surgical site based on the three-dimensional profile of the surface of the surgical site, and generate a visual overlay of at least a portion of the three-dimensional pre-operative scan based on the intersection between the surface of the surgical site and a three-dimensional pre-operative scan of the surface of the surgical site.

## Description

### Technical field

The present disclosure relates to medical imaging and visualization, specifically in the realm of Image-Guided Surgery (IGS). IGS leverages various imaging technologies to provide real-time, 3D visualizations of the patient's anatomy and pathology during surgical procedures.

### Background

Image-Guided Surgery (IGS) systems typically use the working distance (WD) of a surgical microscope as input, visualizing a cross-section of 3D pre-operative data based on a planar tissue surface assumption. Due to the reliance on the assumption of a planar tissue surface, this methodology presents limitations when visualizing non-planar, irregular tissue surfaces, which can result in structures located in different tissue layers (higher or lower) being misrepresented as being on the surface. This can lead to a misrepresentation of the surgical field, wherein structures located in different tissue layers may be visualized as if they were on the surface. Such inaccuracies can be misleading for surgeons during surgical procedures.

Furthermore, the inability of such systems to adapt their visualizations to irregular tissue surfaces prevents them from fully utilizing the available 3D pre-operative data, thus missing out on the potential to provide a more accurate, detailed, and realistic representation of the surgical field. Consequently, these systems may be unable to provide comprehensive surgical guidance, which can compromise the precision of surgical procedures, especially in complex microsurgical scenarios.

There may be a desire for providing an improved concept for image-guided surgery.

### Summary

This desire is addressed by the subject-matter of the independent claims.

Various examples of the present disclosure are based on the finding that the assumption of a planar tissue surface presents limitations when visualizing non-planar, irregular tissue surfaces, which can result in structures located in different tissue layers (higher or lower) being misrepresented as being on the surface. To overcome these limitations, in the proposed concept, instead of using a single distance value, a plurality of distance values are determined for a plurality of points on the surface of the surgical site. From these distances, a three-dimensional profile of the surface is generated and used to generate a visual overlay. To generate the visual overlay pre-operative data is obtained. The pre-operative data is indicative of a three-dimensional pre-operative scan comprising a structure. A determination, of a distance of the surface to the structure is performed. Based on this distance between the three-dimensional profile of the surface and the structure of the three-dimensional pre-operative scan, a more realistic and precise visual overlay can be provided for the purpose of image-guided surgery.

Some aspects of the present disclosure relate to a system for a surgical imaging system. The system comprises one or more processors and one or more storage devices. The system is configured to obtain sensor data from a sensor of the surgical imaging system. The sensor data represents a distance between an objective of a surgical imaging device of the surgical imaging system and a plurality of points of a surface of a surgical site being imaged by the surgical imaging system. The system is configured to determine a three-dimensional profile of the surface of the surgical site based on the sensor data. The system is configured to obtain pre-operative data indicative of a three-dimensional pre-operative scan comprising a structure. The system is configured to determine a distance between the surface of the surgical site and the structure of the three-dimensional pre-operative scan of the surgical site based on the three-dimensional profile of the surface of the surgical site and the preoperative data. The system is configured to generate a visual overlay of at least a portion of the structure of the three-dimensional pre-operative scan based on the distance between the surface of the surgical site and the structure of the three-dimensional pre-operative scan of the surface of the surgical site. This way, a more realistic and precise visual overlay can be generated, which accurately distinguishes between features to be shown (or hidden) at or above the surface and features to be shown (or hidden) below the surface.

In general, the three-dimensional profile of the surface of the surgical site may be used to determine, which features included in the three-dimensional pre-operative scan are at or above the surface of the surgical site and which features are below the surface. For this reason, a cross-section may be determined of the three-dimensional pre-operative scan based on the three-dimensional profile of the surface of the surgical site. In other words, the system may be configured to generate a cross-section of the three-dimensional pre-operative scan based on an intersection between the surface of the surgical site and the three-dimensional pre-operative scan of the surface of the surgical site, and to generate the visual overlay based on the generated cross-section. The cross-section may show the features included in the three-dimensional pre-operative scan according to the current surface of the surgical site.

A major reason for using a three-dimensional profile of the surface instead of a singular working distance is to be able to more accurately determine which features are at or above the surface and which features are below the surface. For example, the system may be configured to determine a first portion of the three-dimensional pre-operative scan to be visualized at or above a surface of the surface of the surgical site and a second portion of the three-dimensional pre-operative scan to be visualized below the surface of the surface of the surgical site based on the distance between the surface of the surgical site and the structure of the three-dimensional pre-operative scan of the surface of the surgical site. This may increase the accuracy of the resulting visual overlay.

To aid the surgeon during the surgical procedure, a clear visual distinction may be made between features at or above the surface and features below the surface. The system may be configured to determine a first portion of the three-dimensional pre-operative scan (e.g., features at or above the surface) and a second portion of the three-dimensional pre-operative scan (e.g., features below the surface) based on the distance between the surface of the surgical site and the structure of the three-dimensional pre-operative scan of the surface of the surgical site. The system may be configured to include the first portion in the visual overlay with a first visualization style. The system may be configured to one of a) include the second portion in the visual overlay with a second visualization style or b) omit the second portion from the visual overlay. This way features at or above and below the surface can be clearly distinguished by the surgeon.

In general, the goal is to more accurately represent the surface of the surgical site. This can be achieved by creating a three-dimensional model of the surface of the surgical site. For example, the three-dimensional profile of the surface of the surgical site may comprise a three-dimensional model of the surface of the surgical site.

For example, there are various options for generating the three-dimensional surface profile based on the sensor data. For example, the sensor data may comprise a three-dimensional surface scan of the surface of the surgical site. A three-dimensional surface scan can be used to accurately model the surface of the surgical site. In general, there are various techniques for performing such a three-dimensional surface scan. For example, the sensor data may comprise a representation of a structured light pattern being emitted by a structured light emitter of the surgical imaging system. Structured light-based techniques have the advantage that a main optical imaging sensor of the respective surgical imaging device can be used for this purpose, in combination with a structured light emitter, without requiring an additional sensor. This may result in a low-cost and low-effort approach. Alternatively, the sensor data may be sensor data of a time-of-flight sensor of the surgical imaging system. Time-of-flight sensors tend to require more hardware (i.e., an emitter and a sensor), but can be used independent of the surgical imaging device, e.g., without disrupting operation of the surgical imaging device. Finally, the sensor data may comprise a plurality of image frames representing a plurality of different focal distances or working distances. On each focal plane or working distance, the system may determine which portions of the image frames are in or out of focus, to determine the three-dimensional profile of the surface of the surgical site. For example, the system may be configured to determine the three-dimensional profile of the surface of the surgical site based on a contrast and/or based on a presence of spatial frequencies above a pre-defined spatial frequency threshold in the respective image frames of the plurality of image frames. Using these techniques (contrast, presence of highfrequency spatial frequencies), a classification of portions of the image frames being in focus/out of focus can be made. This way, no additional hardware may be necessary. However, while capturing the image frames at different focal distances or working distances, the surgical imaging device may not be available to the surgeon.

An aspect of the present disclosure relates to a surgical imaging system comprising the system, the surgical imaging device, and the sensor for providing the sensor data. For example, the surgical imaging device may be one of a microscope and an exoscope. Surgical microscopes and surgical exoscopes are highly relevant for use in surgical procedures.

For example, the sensor for providing the sensor data may be an imaging sensor of the surgical imaging device. This may be the case if a structured light-based technique or a focal plane/working distance-based technique is used.

Alternatively, the sensor for providing the sensor data may be separate from surgical imaging device. This may be the case if a structured light-based technique with a separate sensor or a time-of-flight-based technique is used.

Some aspects of the present disclosure relate to a corresponding method for a surgical imaging system. The method comprises obtaining sensor data from a sensor of the surgical imaging system. The sensor data represents a distance between an objective of a surgical imaging device of the surgical imaging system and a plurality of points of a surface of a surgical site being imaged by the surgical imaging system. The method comprises determining a three-dimensional profile of the surface of the surgical site based on the sensor data. The method comprises obtaining pre-operative data indicative of a three-dimensional pre-operative scan comprising a structure. The method comprises determining a distance between the surface of the surgical site and the structure of the three-dimensional pre-operative scan of the surgical site based on the three-dimensional profile of the surface of the surgical site and the pre-operative data. The method comprises generating a visual overlay of at least a portion of the structure of the three-dimensional pre-operative scan based on the distance between the surface of the surgical site and the structure of the three-dimensional pre-operative scan of the surface of the surgical site.

An aspect of the present disclosure relates to computer program with a program code for performing the above method when the computer program is run on a processor.

### Short description of the Figures

Some examples of apparatuses and/or methods will be described in the following by way of example only, and with reference to the accompanying figures, in which:
- Fig. 1a: shows a block diagram of an example of a system for a surgical imaging system;
- Fig. 1b: shows a schematic diagram of an example of a surgical imaging system;
- Fig. 1c: shows a schematic diagram illustrating the determination of a planar tissue surface;
- Figs. 1d and 1e: shows schematic diagrams illustrating the determination of a three-dimensional profile of a tissue surface;
- Fig. 2: shows a flow chart of an example of a method for a surgical imaging system;
- Figs. 3a and 3b: show an illustration of an IGS being operated under the assumption of a planar tissue surface;
- Figs. 4a and 4b: show an illustration of an IGS being operated based on a three-dimensional tissue scan; and
- Fig. 5: shows a block diagram of an example of a system comprising an imaging device and a computer system.

### Detailed Description

Various examples will now be described more fully with reference to the accompanying drawings in which some examples are illustrated. In the figures, the thicknesses of lines, layers and/or regions may be exaggerated for clarity.

As used herein the term "and/or" includes any and all combinations of one or more of the associated listed items and may be abbreviated as "/".

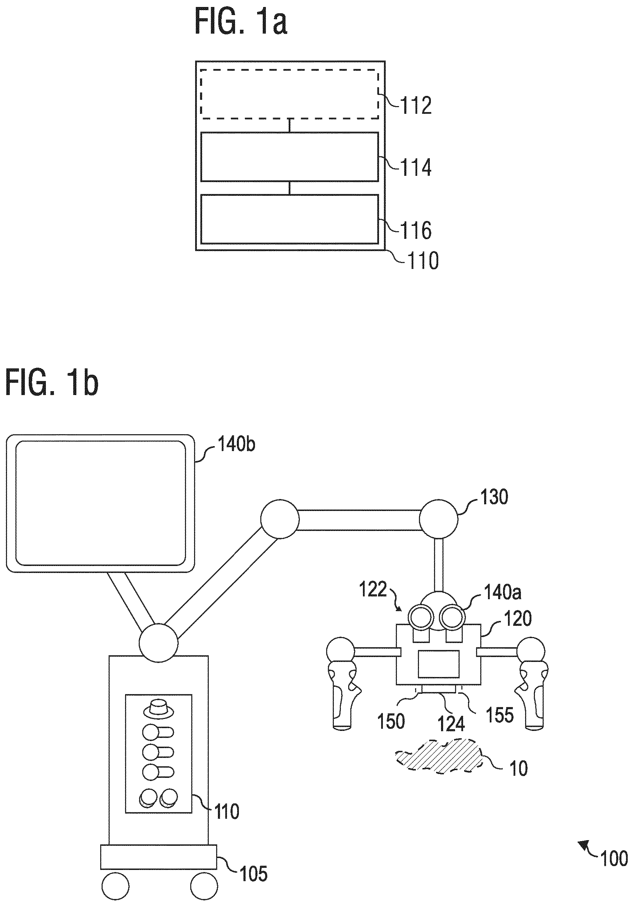

Fig. 1a shows a block diagram of an example of a system 110 for a surgical imaging system 100 (shown in Fig. 1b). The system 110 is a component of the surgical imaging system 100 and may be used to control various aspects of the surgical imaging system 100. In particular, it may be used for acquisition and/or processing of imaging sensor data by an optical imaging sensor of an imaging device 120 of the surgical imaging system or other sensor data, through various means that will be introduced in more detail in the following. In addition, the system 110 may be configured to control additional aspects of the surgical imaging system, e.g., to provide a display signal for various displays of the surgical imaging system.

In general, the system 110 may be considered to be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical imaging system 100 and outside the surgical imaging system 100, such as an optical imaging sensor 122 of the imaging device 120, another sensor 150, or an emitter 155, all shown in Fig. 1b), and with the one or more storage devices 116 (for storing information, such as machine-readable instructions of a computer program being executed by the one or more processors). In general, the functionality of the one or more processors 114 may be implemented by one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical imaging system 100, which comprises various components in addition to the system 110. For example, the surgical imaging system 100 comprises the imaging device 120, and may comprise one or more additional components, such as a robotic arm 130. Fig. 1b shows a schematic diagram of an example of such a surgical imaging system 100, and in particular of a surgical microscope system 100. In the following, the surgical imaging system 100 may also be referred to as surgical microscope system 100, which is a surgical imaging system 100 that comprises a (surgical) microscope as imaging device 120. However, the proposed concept is not limited to such embodiments. The surgical imaging system 100 may be based on various (single or multiple) imaging devices, such as one or more microscopes, one or more endoscopes, one or more exoscopes (also sometimes called an extracorporeal telescope). Exoscopes are camera-based imaging systems, and in particular camera-based 3D imaging systems, which are suitable for providing images of surgical sites with high magnification and a large depth of field. Compared to microscopes, which may be used via oculars, exoscopes are only used via display modalities, such as monitor or a head-mounted display. Accordingly, the surgical imaging system may alternatively be a surgical endoscope system, or a surgical exoscope system. The following illustrations assume that the imaging device 120 is a surgical microscope for use in neurosurgery, and that the surgical imaging system 100 is a surgical microscope system 100 for use in neurosurgery.

Accordingly, the surgical imaging system or surgical microscope system 100 may comprise an imaging device, such as a microscope 120, an endoscope or an exoscope. In general, a microscope, such as the microscope 120, is an optical instrument that is suitable for examining objects that are too small to be examined by the human eye (alone). For example, a microscope may provide an optical magnification of a sample. In the present concept, the optical magnification is (also) provided for an optical imaging sensor. The microscope 120 thus comprises an optical imaging sensor, which is coupled with the system 110. The microscope 120 may further comprise one or more optical magnification components that are used to magnify a view on the sample, such as an objective (i.e., lens). For example, the surgical imaging device or microscope 120 is often also called the "optics carrier" of the surgical imaging system.

There are a variety of different types of surgical imaging devices. If the surgical imaging device is used in the medical or biological fields, the object being viewed through the surgical imaging device may be a sample of organic tissue, e.g., arranged within a petri dish or present in a part of a body of a patient. In various examples presented here, the imaging device 120 may be a microscope of a surgical microscope system, i.e., a microscope that is to be used during a surgical procedure, such as a neurosurgical procedure or an ophthalmological procedure (i.e., eye surgery). Accordingly, the object being viewed through the surgical imaging device (that is shown in the field of view of the surgical imaging device) and shown in the digital view generated by based on the imaging sensor data provided by the (optional) optical imaging sensor, may be a sample of organic tissue of a patient, and may be in particular be the surgical site that the surgeon operates on during the surgical procedure, e.g., the brain or the eye. However, the proposed concept is also suitable for other types of surgery, such as cardiac surgery.

Fig. 1b shows a schematic diagram of an example of a surgical imaging system 100, and in particular of a surgical microscope system 100 for use in neurosurgery, comprising the system 110 and a microscope 120. The surgical microscope system 100 shown in Fig. 1b comprises a number of optional components, such as a base unit 105 (comprising the system 110) with a (rolling) stand, ocular displays 140a that are arranged at the microscope 120, an auxiliary display 140b that is arranged at the base unit, the robotic arm 130 that holds the microscope 120 in place, and which is coupled to the base unit 105 and to the microscope 120. In general, these optional and non-optional components may be coupled to the system 110 which may be configured to control and/or interact with the respective components.

In the proposed concept, the system is used for the purpose of performing image-guided surgery. Image-guided surgery (IGS) is a surgical tool in which pre-operative and intraoperative imaging is used to guide the surgeon and to track the exact location of surgical tools in relation to the patient's anatomy during an operation. This technique is designed to help surgeons navigate through the body in a more precise manner, particularly in areas that are difficult to view or are too risky to explore through traditional surgical approaches. There are several types of imaging modalities frequently used in image-guided surgery, including Computed Tomography (CT) scans and Magnetic Resonance Imaging (MRI). By integrating the images generated based on the pre-operative scan with the digital view on the surgical site, surgeons are provided with a continuous visual guide on a screen that shows them the location of three-dimensional anatomical features of interest.

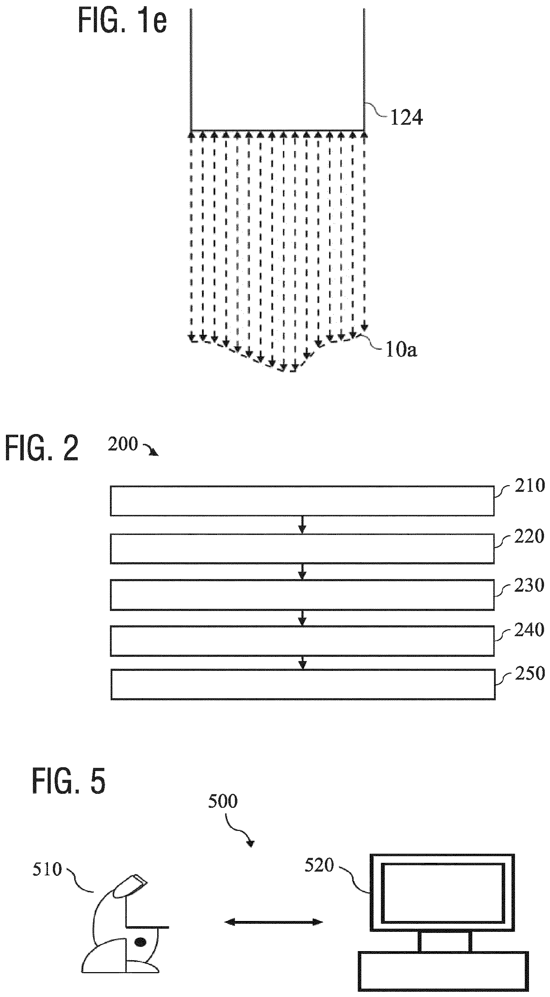

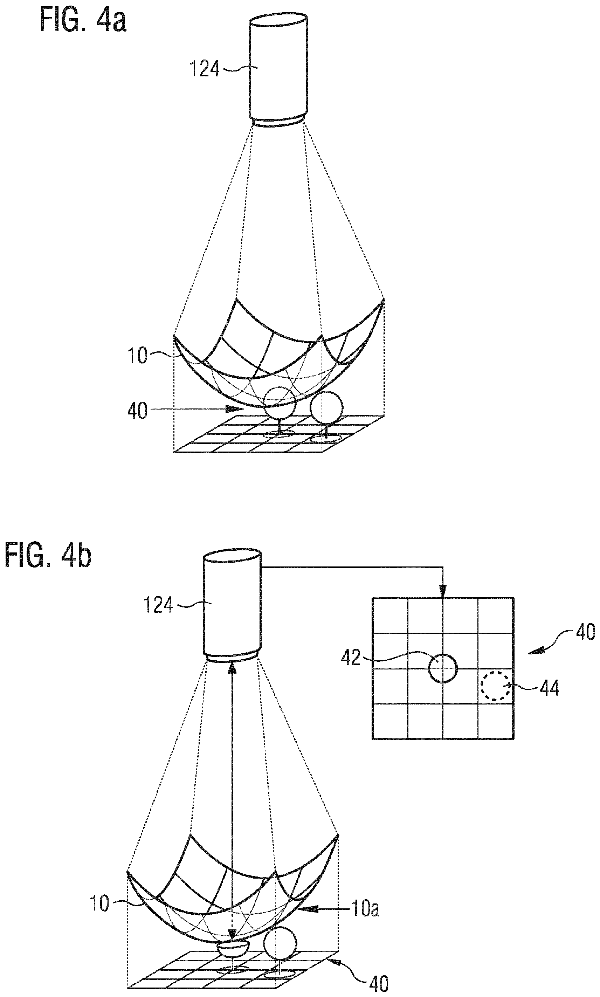

However, the present disclosure does not relate to the techniques used for performing the pre-operative scans or registering the pre-operative scan with the surgical site, but to the technique of overlaying a visual representation of the pre-operative scan over the digital view of the surgical site. Therefore, the system 110 is configured to obtain sensor data from a sensor of the surgical imaging system. The sensor data represents a distance between an objective 124 of a surgical imaging device 120 of the surgical imaging system and a plurality of points of a surface 10 of a surgical site being imaged by the surgical imaging system. The system 110 is configured to determine a three-dimensional profile 10a (shown in Fig. 1e and 4b) of the surface of the surgical site based on the sensor data. The system 110 is configured to obtain pre-operative data indicative of a three-dimensional pre-operative scan comprising a structure (e.g., a structure 32 or 34 as described below). The system 110 is configured to determine a distance between the surface of the surgical site and the structure of the three-dimensional pre-operative scan of the surgical site based on the three-dimensional profile of the surface of the surgical site and the pre-operative data. The system 110 is configured to generate a visual overlay of at least a portion of the structure of the three-dimensional pre-operative scan based on the distance between the surface of the surgical site and the structure of the three-dimensional pre-operative scan of the surface of the surgical site. For example, the surgical imaging system 100 may comprise the system 110, the surgical imaging device 120, and the sensor for providing the sensor data. For example, as shown in Fig. 1b, the sensor for providing the sensor data may be an optical imaging sensor 122 of the surgical imaging device 120. Alternatively, as also shown in Fig. 1b, a sensor 150 for providing the sensor data may be separate from surgical imaging device.

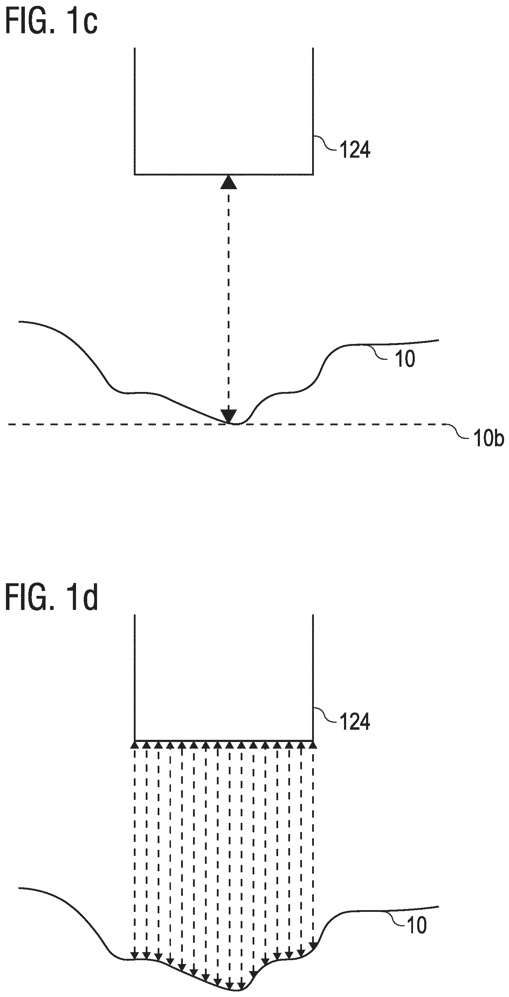

The course of action taken in the proposed concept is illustrated in Figs. 1c to 1e. In Fig. 1c, the commonly used approach is shown. Fig. 1c shows a schematic diagram illustrating the determination of a planar tissue surface. As shown in Fig. 1c, in other surgical imaging systems, an assumption is made for the purpose of image-guided surgery: The assumption is that the surgical site is essentially flat and can therefore be represented by a plane 10b that is based on the working distance between the objective 124 and the part of the surgical site that is in focus. As is evident from Fig. 1c, this assumption is not always useful, as many surgical sites are surgical cavities with large degrees of unevenness.

In Figs. 1d and 1e, the proposed approach is illustrated. Figs. 1d and 1e shows schematic diagrams illustrating the determination of a three-dimensional profile of a tissue surface. Please note that Figs. 1d and 1e only illustrate the proposed approach of generating a three-dimensional profile in only two dimensions to simplify the drawing. The proposed approach, however, extends the approach illustrated in these figures into three dimensions. Three-dimensional illustrations of the concept are shown in Figs. 4a and 4b, for example. As can be seen in Figs. 1d and 1e, as well as 4a and 4b, instead of the assumption of a planar surface, the surface of the surgical site is accurately modelled in three dimensions. In Fig. 1d, the plurality of distances between the objective 124 and the surface 10 of the surgical site are illustrated. In Fig. 1e, the resulting three-dimensional (or rather two-dimensional, in case of the illustration) profile 10a of the surface of the surgical site is shown. In effect, the three-dimensional profile of the surface of the surgical site may comprise a three-dimensional model of the surface of the surgical site. A three-dimensional model of a surface is a mathematical or digital representation of a surface's form in three dimensions. Unlike 2D models, which only include width and height, three-dimensional models also incorporate depth. The three-dimensional profile of the surface of the surgical site may comprise a three-dimensional topology of the surgical site. It may represent the distances between the plurality of points on the surface of the surgical site and the objective 124 of the surgical imaging device. For example, the three-dimensional profile of the surface of the surgical site may be a three-dimensional point cloud of the surgical site.

In the proposed concept, the three-dimensional profile of the surface of the surgical site is determined based on the sensor data, with the sensor data representing a distance between the objective 124 of the surgical imaging device 120 of the surgical imaging system and the plurality of points of the surface 10 of a surgical site being imaged by the surgical imaging system. This can be done by scanning the surface of the surgical site using a 3D scanning technique. In other words, the sensor data may comprise a three-dimensional surface scan of the surface of the surgical site. There are a number of different 3D scanning techniques that can be used for this purpose.

For example, structed light-based scanning may be used. Structured light 3D scanning is an optical method used to capture the three-dimensional profile of objects using projected light patterns and a camera, such as the optical imaging sensor 122 of the surgical imaging device 120, or another camera sensor 150. The process involves projecting a known pattern of light, e.g., stripes or a grid, onto a surface and using a camera to take images of the distorted pattern from different angles. For example, the surgical imaging system may comprise a structured light emitter 155 (shown in Fig. 1b) (usually a digital projector or laser) to project a known pattern onto the surface of the surgical site. As the light pattern conforms to the geometry of the surgical site, it becomes distorted. These distortions provide clues about the depth and contours of the surface. The optical imaging sensor 122 or other camera sensor 150 may then be used to capture image frames of the distorted light pattern as it appears on the surface of the surgical site. For example, the sensor data may comprise a representation of a structured light pattern being emitted by the structured light emitter 155 of the surgical imaging system. The system may be configured to process the representation of the structured light pattern to calculate the position of the plurality of points on the surface of the surgical site in three-dimensional space, based on the known geometry of the pattern ad based on the angles of the camera and projector, and the way the pattern deforms on the obj ect.

Alternatively, a Time-of-Flight (ToF) sensor may be used to determine the three-dimensional profile of the surgical site. Accordingly, the sensor data may be sensor data of a time-of-flight sensor 150 of the surgical imaging system. Accordingly, the surgical imaging system may comprise a corresponding emitter 155 for the time-of-flight sensor 150. The time-of-flight sensor may similarly provide a point-cloud of the surgical site, which the system uses to determine the three-dimensional profile of the surgical site.

Another technique is to capture a plurality of image frames at a plurality of different focal planes or at a plurality of different working distances. In other words, the sensor data may comprise a plurality of image frames representing a plurality of different focal distances or working distances. Any portion of the respective image frame that is in focus at a given focal plane or working distance is assumed have its surface (depth-wise) at the respective focal plane or working distance. When using this technique, the image sharpness is used to determine which portions of the image frame are in focus at a given focal distance or working distance. The sharpness of the different portions may be determined based on the contrast of the respective images and/or based on the proportion of high spatial frequencies in the respective image frames. For example, the system may be configured to determine the three-dimensional profile of the surface of the surgical site based on a contrast and/or based on a presence of spatial frequencies above a pre-defined spatial frequency threshold in the respective image frames of the plurality of image frames, e.g., by determining the surface of the portions of the surgical site to be at the focal plane or working distance at which the respective portions of the surgical site appear sharp in the respective image frame representing the focal plane or working distance.

For example, the system may be configured to determine the contrast of the portions (e.g., blocks of pixels) of the respective image frames, e.g., by determining the ratio of the standard deviation and the mean value of the pixels of the image, or by doing kernel-based comparisons between individual pixels and their neighbors (i.e., adjacent pixels). The sharper a portion of an image appears, the higher the contrast of the image generally is.

The system may also be configured to determine the distribution of spatial frequencies of the respective portions (e.g., blocks of pixels) of the imaging sensor data, e.g., by performing a 2D Fourier transform of the respective image frames. The higher the proportion of high spatial frequencies in the respective portions of the image frames, the more fine-grained structures are visible in the portion of the image frames, which is the case if the respective portions of the image frame containing the fine-grained structures are perceived as sharp. By determining the integral of the portion of the distribution of the spatial frequencies above a pre-defined frequency, a quantitative measure can be determined that can be used to compare the presence of fine-grained structure across different image frames, e.g., to compare the sharpness of two corresponding portions of a field of view in different image frames.

Once the three-dimensional profile of the surface of the surgical site is determined, it can be used to determine the distance between the surface of the surgical site and a structure of the three-dimensional pre-operative scan of the surgical site. This is particularly relevant to distinguish between three-dimensional features / structures that are to be shown at or above the surface of the surgical site and three-dimensional features / structures that are to be shown below the surface of the surgical site (or not at all). In effect, the distance between the surface of the surgical site and the structure of the three-dimensional pre-operative scan of the surgical site may be used to "cut open" the three-dimensional pre-operative scan, to visualize which anatomical features are at or below the surface of the surgical site.

For example, the system may be configured to generate the visual overlay such that the structure of the three-dimensional pre-operative scan is visualized differently for different determined distances. That is, the structure of the three-dimensional pre-operative scan can be visualized differently depending on the distance to the surface of the surgical site. For example, the structure may be included in the visual overlay with a first visualization style, e.g., with a first bolder (i.e., stronger lines) or brighter (i.e., lines with a brighter tint or more prominent color) visualization style when the structure intersects the surface of the surgical site (i.e., the distance is 0). For example, the structure may be included in the visual overlay with a second visualization style, (e.g., with a second more subdued visualization style, with thinner lines, lines with a darker tint, a less prominent color or dotted lines) when the structure does not intersect the surface of the surgical site (i.e., the distance is greater than 0).

For example, the system may be configured to generate a cross-section of the three-dimensional pre-operative scan based on an intersection between the surface of the surgical site and the three-dimensional pre-operative scan of the surface of the surgical site. In other words, the system may be configured to determine, where the surface of the surgical site intersects with the three-dimensional pre-operative scan of the surgical site. The visual overlay may then be generated based on the generated cross-section, by showing the three-dimensional features included in the three-dimensional pre-operative scan according to the cross-section, e.g., "cut through" the three-dimensional features included in the three-dimensional pre-operative scan at the cross-section.

To aid in the interpretation of the three-dimensional pre-operative scan, a distinction may be made between features that are located at or above the surface and features that are located below the surface. Accordingly, the system may be configured to determine a first portion 42 (shown in Fig. 4b) of the three-dimensional pre-operative scan to be visualized at or above a surface of the surface of the surgical site and a second portion 44 (also shown in Fig. 4b) of the three-dimensional pre-operative scan to be visualized below the surface of the surface of the surgical site based on the distance between the surface of the surgical site and the structure of the three-dimensional pre-operative scan of the surface of the surgical site. For example, the first portion of the three-dimensional pre-operative scan may be three-dimensional features/structures included in the three-dimensional pre-operative scan that are at an intersection defined by the three-dimensional profile of the surface of the surgical site, and thus intersected by the cross-section of the three-dimensional pre-operative scan. The second portion of the three-dimensional pre-operative scan may be three-dimensional features/structures included in the three-dimensional pre-operative scan that are below an intersection defined by the three-dimensional profile of the surface of the surgical site, i.e., further away from the objective 124 than the surface of the surgical site. The system may be configured to determine such a first portion 42 of the three-dimensional pre-operative scan and a second portion 44 of the three-dimensional pre-operative scan based on the intersection between the surface of the surgical site and the three-dimensional pre-operative scan of the surface of the surgical site. These portions may be visualized differently in the visual overlay. For example, the first portion in the visual overlay may be included in the visual overlay with a first visualization style, e.g., with a first bolder (i.e., stronger lines) or brighter (i.e., lines with a brighter tint or more prominent color) visualization style. The second potion may be included with a second visualization style (e.g., with a second more subdued visualization style, with thinner lines, lines with a darker tint or a less prominent color etc.) or omitted from the visual overlay.

The system generates the visual overlay of at least a portion of the three-dimensional pre-operative scan (e.g., a portion according to a current field of view, a portion at or above the surface, and/r or a portion at or below the surface) based on the distance between the surface of the surgical site and the three-dimensional pre-operative scan of the surface of the surgical site, e.g., a described above. For example, at least some aspects of the generation of the visual overlay may be performed by a separate image-guided surgery system (not shown). If such a separate image-guided surgery system is used, the three-dimensional profile of the surface of the surgical site may be provided as input to the separate image-guided surgery system, and the visual overlay may be obtained from the image-guided surgery system. In this case, some of the operations described above that are related to the generation of the cross-section of the three-dimensional pre-operative scan and of the visual overlay may be performed by the image-guided surgery system. The system may be configured to combine the visual overlay with (e.g., to overlay the visual overlay over) a digital view of the surgical site that is based on imaging sensor data of the optical imaging sensor 122 of the surgical imaging device 120. The system may be configured to provide a display signal with the combined digital view and visual overlay, e.g., to at least one of the display device(s) 140a, 140b of the surgical imaging system 100.

For example, the display signal may be a signal for driving (e.g., controlling) the respective display device 140a; 140b. For example, the display signal may comprise video data and/or control instructions for driving the display device. For example, the display signal may be provided via one of the one or more interfaces 112 of the system. Accordingly, the system 110 may comprise a video interface 112 that is suitable for providing the display signal to the display device 140a; 140b of the surgical imaging system 100.

In the proposed surgical imaging system, the optical imaging sensor is used to provide the imaging sensor data, and, optionally, the sensor data. Accordingly, the optical imaging sensor, which may be part of the surgical imaging device 120 (e.g., of the microscope) may be configured to generate the imaging sensor data and/or sensor data. For example, the optical imaging sensor of the surgical imaging device 120 may comprise or be an APS (Active Pixel Sensor) - or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging. The system 110 may be configured to obtain (i.e., receive or read out) the imaging sensor data and/or sensor data from the optical imaging sensor. The imaging sensor data and/or sensor data may be obtained by receiving the imaging sensor data and/or sensor data from the optical imaging sensor (e.g., via the interface 112), by reading the imaging sensor data and/or sensor data out from a memory of the optical imaging sensor (e.g., via the interface 112), or by reading the imaging sensor data and/or sensor data from a storage device 116 of the system 110, e.g., after the imaging sensor data and/or sensor data has been written to the storage device 116 by the optical imaging sensor or by another system or processor. The system 110 may be configured to obtain (i.e., receive or read out) the pre-operative data from the optical imaging sensor and/or an external storage device. The pre-operative data may be obtained by receiving the pre-operative data from the optical imaging sensor (e.g., via the interface 112), by reading the imaging sensor data out from a memory of the optical imaging sensor (e.g., via the interface 112), or by reading the imaging sensor data from a storage device 116 of the system 110, e.g., after the pre-operative data has been written to the storage device 116 by the optical imaging sensor or by another system or processor, e.g., comprising an external storage device.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system 110 and surgical imaging system 100 are mentioned in connection with the proposed concept or one or more examples described above or below (e.g., Fig. 2 to 5). The system 110 and surgical imaging system 100 may comprise one or more additional optional features corresponding to one or more aspects of the proposed concept, or one or more examples described above or below.

Fig. 2 shows a flow chart of an example of a correspond method for a surgical imaging system, e.g., for the surgical imaging system 100 discussed in connection with Figs. 1a to 1e. The method comprises obtaining 210 sensor data from a sensor of the surgical imaging system, the sensor data representing a distance between an objective of a surgical imaging device of the surgical imaging system and a plurality of points of a surface of a surgical site being imaged by the surgical imaging system. The method comprises determining 220 a three-dimensional profile of the surface of the surgical site based on the sensor data. The method 200 comprises determining 230 a distance between the surface of the surgical site and the structure of the three-dimensional pre-operative scan of the surgical site based on the three-dimensional profile of the surface of the surgical site and the pre-operative data. The method 200 comprises generating 240 a visual overlay of at least a portion of the structure of the three-dimensional pre-operative scan based on the distance between the surface of the surgical site and the structure of the three-dimensional pre-operative scan of the surface of the surgical site. The method 200 comprises generating 250 a visual overlay of at least a portion of the structure of the three-dimensional pre-operative scan based on the distance between the surface of the surgical site and the structure of the three-dimensional pre-operative scan of the surface of the surgical site.

For example, the method 200 may be implemented by the surgical imaging system introduced in connection with one of the Figs. 1a to 1e. Features introduced in connection with the (surgical) imaging system of Figs. 1a to 1e may likewise be included in the corresponding method 200.

More details and aspects of the method are mentioned in connection with the proposed concept, or one or more examples described above or below (e.g., Fig. 1a to 1e, 3a to 5). The method may comprise one or more additional optional features corresponding to one or more aspects of the proposed concept, or one or more examples described above or below.

Various examples of the present disclosure relate to a concept for accurate 3D surface-adapted visualization in image-guided surgery, in short 3D-adaptive IGS.

The proposed concept particularly addresses the use of IGS in microsurgical procedures, where precision is of utmost importance. By improving the accuracy of visualized data, proposed concept may significantly enhance surgical outcomes in these high-precision environments.

A major insight used in the proposed concept is the integration of 3D surface scanning capabilities within the Image-Guided Surgery (IGS) system to allow for more accurate visualization of the patient's anatomy during surgery. The proposed concept addresses the issues associated with the planar tissue surface assumption typically used in IGS systems by incorporating a method to scan and generate an accurate 3D representation of the actual, non-planar tissue surface. This surface data can then be used to inform the IGS system, enabling it to present a surface-adapted cross-section of the pre-operative 3D structures. Whether the structures are located in higher or lower layers than the surface, they can be accurately represented in their actual locations rather than being incorrectly shown as being on the surface. By offering a more accurate visualization that adheres closely to the actual tissue topology, the system provides surgeons with more precise guidance during procedures. This in turn helps to reduce the risks of surgical inaccuracies, which may enhance patient safety and potentially lead to improved surgical outcomes.

The proposed concept directly addresses a potential pain point experienced by surgeons who rely on Image-Guided Surgery (IGS) systems for accurate visualization during surgical procedures: The inaccurate depiction of structures located at different tissue layers due to the assumption of a planar tissue surface By providing a more accurate, surface-adapted visualization of the surgical field, this invention allows surgeons to operate with greater precision and confidence. This not only enhances the surgeon's ability to perform safe and effective procedures, but it can also reduce the risk of misinterpretation of the IGS data of the surgical field, thereby directly addressing the above-mentioned pain point.

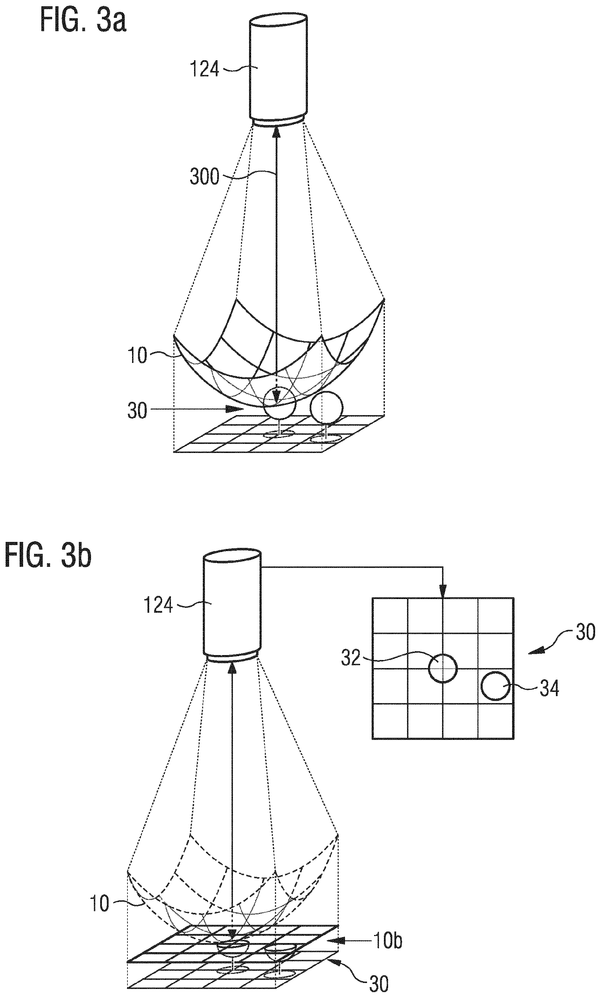

Figs. 3a and 3b show an illustration of an IGS being operated under the assumption of a planar tissue surface. Fig. 3a shows an objective 124 of a surgical microscope, the tissue surface 10 and segmented pre-operative 3D structures 30 of a three-dimensional pre-operative scan. Fig. 3a further shows a working distance 300 between the objective 124 and the center of the tissue surface 10. Fig. 3a shows an illustration of the IGS addressing the surgeon's need for 3D pre-operative anatomical and pathological information. Fig. 3b shows a demonstration of the IGS visualizing segmented pre-operative 3D structures based on a flat plane cross-section 10b at the microscope's working distance. The non-planar nature of actual tissue surfaces can lead to inaccuracies in the visualized depth of these pre-operative 3D structures. For example, in Fig. 3b, on the top right side, a schematic drawing of the resulting tissue image that is augmented with pre-operative 3D structures is shown. 3D structure 32 of the pre-operative scan 30 is depicted correctly, as the structure is depicted at the tissue surface. 3D structure 34 is misrepresented as being on surface level, while its actual location is at a deeper tissue layer.

Figs. 4a and 4b show an illustration of an IGS being operated based on a three-dimensional tissue scan. Fig. 4a depicts a surgical microscope (i.e., the objective 124 thereof) with 3D surface scanning capability, which provides accurate tissue surface information of the surface 10 of the surgical site to the IGS for correct cross-section calculation. Fig. 4a further shows the segmented pre-operative 3D structures of the 3D pre-operative scan 40. Fig. 4b showcases how visualized data accurately matches the actual tissue surface, resulting in a more precise visualization. In Fig. 4b, the surface-adapted section 10a (i.e., the 3D profile of the surgical site) is shown, together with segmented pre-operative 3D structures of the 3D pre-operative scan 40. On the top right of Fig. 4b, a schematic drawing of the tissue image augmented with the pre-operative 3D structures is shown. In Fig. 4b, 3D structure 42 is correctly depicted at the tissue surface, and 3D structure 44 (shown with a dotted outline) accurately indicates that structure being in a deeper layer, not on the surface.

The 3D surface scan of the tissue can be achieved through a number of different imaging techniques, each adaptable to the unique requirements of surgical microscopes. A common method is structured light scanning, where a known light pattern is projected onto the surface and analyzed for deformations to generate a 3D model. Time-of-flight scanning is another method that measures the time a pulse of light takes to travel to the surface and back. Specifically for surgical microscopes, a technique called depth from focus or focus stacking can be used. This involves sequentially adjusting the microscope's working distance, capturing an image at each step, and analyzing each image to identify at which working distance each area is best focused. This information can then be used to create a detailed 3D map of the tissue surface. The choice of scanning method may depend on a variety of factors including the required resolution, the nature of the tissue being scanned, and the specific needs and constraints of the surgical procedure. Each of these methods can be integrated into the surgical microscope (or more generally surgical imaging device) to provide the 3D surface scanning capability of this invention.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an imaging device, such as a microscope or exoscope, comprising a system as described in connection with one or more of the Figs. 1a to 4b. Alternatively, a imaging device, such as a microscope or exoscope, may be part of or connected to a system as described in connection with one or more of the Figs. 1a to 4b. Fig. 5 shows a schematic illustration of a system 500 configured to perform a method described herein. The system 500 comprises an imaging device 510 and a computer system 520. The imaging device 510 is configured to take images and is connected to the computer system 520. The computer system 520 is configured to execute at least a part of a method described herein. The computer system 520 may be configured to execute a machine learning algorithm. The computer system 520 and imaging device 510 may be separate entities but can also be integrated together in one common housing. The computer system 520 may be part of a central processing system of the imaging device 510 and/or the computer system 520 may be part of a subcomponent of the imaging device 510, such as a sensor, an actor, a camera or an illumination unit, etc. of the imaging device 510.

The computer system 520 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a imaging device or a imaging device component (e.g., camera) or any other type of processor or processing circuit. Other types of circuits that may be included in the computer system 520 may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference Signs

- 10: Surface of a surgical site
- 30: Pre-operative scan
- 32,34: 3D structures of a pre-operative scan
- 40: Pre-operative scan
- 42,44: 3D structures of a pre-operative scan
- 10a: Three-dimensional profile of the surface of the surgical site
- 10b: Planar representation of the surface of the surgical site
- 100: Surgical imaging system
- 110: System
- 112: One or more interfaces
- 114: One or more processors
- 116: One or more storage devices
- 120: Surgical imaging device
- 122: Optical imaging sensor
- 124: Objective
- 130: Robotic arm
- 140a: Ocular displays
- 140b: Auxiliary display
- 150: Sensor
- 155: Emitter
- 210: Obtaining sensor data
- 220: Determining a three-dimensional profile of a surface of a surgical site
- 230: Obtaining pre-operative data
- 240: Determining a distance
- 250: Generating a visual overlay
- 300: Working distance
- 500: System
- 510: Imaging device
- 520: Computer system

## Claims

1. A system (110) for a surgical imaging system (100), the system comprising one or more processors and one or more storage devices, wherein the system is configured to:
obtain sensor data from a sensor (150; 122) of the surgical imaging system, the sensor data representing a distance between an objective (124) of a surgical imaging device (120) of the surgical imaging system and a plurality of points of a surface (10) of a surgical site being imaged by the surgical imaging system;
determine a three-dimensional profile (10a) of the surface of the surgical site based on the sensor data;
obtain pre-operative data indicative of a three-dimensional pre-operative scan comprising a structure;
determine a distance between the surface of the surgical site and the structure of the three-dimensional pre-operative scan (40) of the surgical site based on the three-dimensional profile of the surface of the surgical site and the pre-operative data; and
generate a visual overlay of at least a portion of the structure of the three-dimensional pre-operative scan based on the distance between the surface of the surgical site and the structure of the three-dimensional pre-operative scan of the surface of the surgical site.

2. The system according to claim 1, wherein the system is configured to generate a cross-section of the three-dimensional pre-operative scan based on an intersection between the surface of the surgical site and the three-dimensional pre-operative scan of the surface of the surgical site, and to generate the visual overlay based on the generated cross-section.

3. The system according to one of the claims 1 or 2, wherein the system is configured to determine a first portion (42) of the three-dimensional pre-operative scan to be visualized at or above a surface of the surface of the surgical site and a second portion (44) of the three-dimensional pre-operative scan to be visualized below the surface of the surface of the surgical site based on the distance between the surface of the surgical site and the structure of the three-dimensional pre-operative scan of the surface of the surgical site.

4. The system according to one of the claims 1 to 3, wherein the system is configured to determine a first portion (42) of the three-dimensional pre-operative scan and a second portion (44) of the three-dimensional pre-operative scan based on the distance between the surface of the surgical site and the structure of the three-dimensional pre-operative scan of the surface of the surgical site, and to include the first portion in the visual overlay with a first visualization style and a) include the second portion in the visual overlay with a second visualization style or b) omit the second portion from the visual overlay.

5. The system according to one of the claims 1 to 3, wherein the three-dimensional profile of the surface of the surgical site comprises a three-dimensional model of the surface of the surgical site.

6. The system according to one of the claims 1 to 5, wherein the sensor data comprises a three-dimensional surface scan of the surface of the surgical site.

7. The system according to one of the claims 1 to 6, wherein the sensor data comprises a representation of a structured light pattern being emitted by a structured light emitter of the surgical imaging system.

8. The system according to one of the claims 1 to 6, wherein the sensor data is sensor data of a time-of-flight sensor of the surgical imaging system.

9. The system according to one of the claims 1 to 6, wherein the sensor data comprises a plurality of image frames representing a plurality of different focal distances or working distances.

10. The system according to claim 9, wherein the system is configured to determine the three-dimensional profile of the surface of the surgical site based on a contrast and/or based on a presence of spatial frequencies above a pre-defined spatial frequency threshold in the respective image frames of the plurality of image frames.

11. A surgical imaging system (100) comprising the system according one of the claims 1 to 10, the surgical imaging device (120), and the sensor (150; 122) for providing the sensor data.

12. The surgical imaging system according to claim 11, wherein the sensor for providing the sensor data is an imaging sensor (122) of the surgical imaging device (120), or wherein the sensor (150) for providing the sensor data is separate from surgical imaging device.

13. The surgical imaging system according to one of the claims 11 or 12, wherein the surgical imaging device is one of a microscope and an exoscope.

14. A method for a surgical imaging system, the method comprising:
obtaining (210) sensor data from a sensor of the surgical imaging system, the sensor data representing a distance between an objective of a surgical imaging device of the surgical imaging system and a plurality of points of a surface of a surgical site being imaged by the surgical imaging system;
determining (220) a three-dimensional profile of the surface of the surgical site based on the sensor data;
obtaining (230) pre-operative data indicative of a three-dimensional pre-operative scan comprising a structure;
determining (240) a distance between the surface of the surgical site and the structure of the three-dimensional pre-operative scan of the surgical site based on the three-dimensional profile of the surface of the surgical site pre-operative data; and
generating (250) a visual overlay of at least a portion of the structure of the three-dimensional pre-operative scan based on the distance between the surface of the surgical site and the structure of the three-dimensional pre-operative scan of the surface of the surgical site.

15. Computer program with a program code for performing the method according to claim 14 when the computer program is run on a processor.
